# EUROPEAN PATENT APPLICATION

(11) **EP 4 851 294 A1**
(43) Date of publication of application: **09.12.2026**
(21) Application number: 25748315.6
(22) Date of filing: 16.01.2025
(51) Int. Cl.: C12N 15/864, A61K 35/76, A61K 35/761, A61K 48/00, A61P 25/00, A61P 25/16, A61P 25/18, A61P 25/28, A61P 43/00, C12N 5/10, C12N 5/0793, C12N 7/01, C12N 15/12, C12N 15/39, C12N 15/113

(54) **IMPROVED MICROGLIA-TARGETING AAV VECTOR**

(30) Priority: 31.01.2024 JP 2024013394
(71) Applicant: National University Corporation Gunma University, Maebashi-shi, Gunma 371-8510 (JP)
(72) Inventor: HIRAI, Hirokazu, Maebashi-shi, Gunma 371-8510 (JP); KONNO, Ayumu, Maebashi-shi, Gunma 371-8510 (JP); AOKI, Ryo, Maebashi-shi, Gunma 371-8510 (JP)
(74) Representative: Gill Jennings & Every LLP
(86) International application number: PCT/JP2025/001061
(87) International publication number: WO 2025/164317

(57) **Abstract**

The present inventors have discovered an AAV vector that enables microglia-specific gene expression over a long period of time and that can be used for the treatment of brain diseases. Specifically, it has been found that a target gene can be expressed in a microglia-specific manner by using the following vector: an adeno-associated virus (AAV) vector for gene expression in microglia, the vector comprising an expression unit that comprises: an Iba1 (ionized calcium-binding adaptor molecule 1) promoter; a sequence complementary to miR-9; a sequence complementary to miR-129; and a woodchuck hepatitis virus post-transcriptional regulatory element (WPRE) gene, wherein the sequence complementary to miR-9 and the sequence complementary to miR-129 are placed upstream of the WPRE gene, or the sequence complementary to miR-9 and the sequence complementary to miR-129 are placed both upstream and downstream of the WPRE gene.

## Description

### TECHNICAL FIELD

The present invention relates to an adeno-associated virus vector which has a high specificity for microglia, and which enables efficient gene expression in microglia.

### BACKGROUND ART

Microglia, which are immunocompetent cells in the central nervous system, have been clarified to be involved in memory and learning as well as the onset of neuropsychiatric diseases, in recent years. Since microglia are cells that fight viruses that have entered the brain, gene transfer into microglia using a viral vector has been extremely difficult.

In 2019, the present inventors succeeded in enabling gene expression in a manner selective to microglia of mice, using an adeno-associated virus (AAV) vector (Patent Document 1). The AAV disclosed in Patent Document 1 incorporates a promoter for a macrophage/microglia-specific molecule, Iba1 (ionized calcium-binding adaptor molecule 1), and sequences complementary to microRNAs (miRs).

The mouse Iba1 promoter used in Patent Document 1 has a certain degree of promoter activity in neurons as well. Therefore, in Patent Document 1, for example, a sequence comprising four repeats of each of a sequence complementary to miR-9 (miR-9.T) and a sequence complementary to miR-129-2-3p (miR-129-2-3p.T), which miRs are endogenously expressed in neurons but not in microglia, was inserted downstream of a woodchuck hepatitis virus post-transcriptional regulatory element (WPRE) gene, in order to inhibit gene expression in neurons. The AAV vector injected into the brain was transcribed in both microglia and neurons. In neurons, however, miR-9 and miR-129-2-3p endogenously expressed therein bound to their complementary sequences contained in the transcribed mRNA and degraded the mRNA. This enabled microglia-selective gene expression at a time point one week after the injection of the AAV vector. However, the expression of the introduced genes was observed also in neurons, three weeks after the injection of the AAV vector. Such unwanted gene expression in neurons was observed markedly in cerebral cortex, in particular.

### RELATED ART DOCUMENT

### PATENT DOCUMENT

Patent Document 1: WO 2021/177116

### SUMMARY OF THE INVENTION

### PROBLEMS TO BE SOLVED BY THE INVENTION

An object of the present invention is to provide an AAV vector which enables microglia-specific gene expression over a long period of time, and which can be used for the treatment of brain diseases.

### MEANS FOR SOLVING THE PROBLEMS

In Patent Document 1, the present inventors have disclosed an AAV vector for selective gene expression in microglia, which vector comprises an Iba1 promoter and sequences complementary to miRs, and also comprises a WPRE gene. However, the order of arrangement of the sequences complementary to miRs and the WPRE gene in the AAV vector is not specified in Patent Document 1, and only the vector in which the sequences complementary to miRs are placed downstream of the WPRE gene is disclosed in Examples.

The present inventors have made intensive studies to solve the above-mentioned problems. As a result, the inventors have discovered that, in a microglia-specific AAV vector comprising an Iba1 promoter, sequences complementary to miRs and a WPRE gene, it is possible to increase the specificity of gene expression to microglia and to enhance the efficiency of gene expression as compared with a conventional AAV vector, by placing the sequences complementary to miRs upstream of the WPRE, or both upstream and downstream of the WPRE, thereby completing the present invention.

Specifically, the present invention is as follows.
[1] An adeno-associated virus (AAV) vector for gene expression in microglia, the vector comprising an expression unit that comprises:
   an Iba1 (ionized calcium-binding adaptor molecule 1) promoter,
   a sequence complementary to miR-9,
   a sequence complementary to miR-129, and
   a woodchuck hepatitis virus post-transcriptional regulatory element (WPRE) gene,
   wherein:
      the sequence complementary to miR-9 and the sequence complementary to miR-129 are placed upstream of the WPRE gene; or
      the sequence complementary to miR-9 and the sequence complementary to miR-129 are placed both upstream and downstream of the WPRE gene.
[2] The vector according to [1], further comprising a target gene placed under the control of the Iba1 promoter.
[3] The vector according to [2], wherein the target gene is a green fluorescent protein (GFP) gene.
[4] The vector according to any one of [1] to [3], wherein the vector comprises multiple repeats of each of the sequence complementary to miR-9 and the sequence complementary to miR-129.
[5] The vector according to any one of [1] to [4], wherein the vector comprises a base sequence of any one of SEQ ID NOs. 1 to 4, or a base sequence having a sequence that is at least 90% identical to the base sequence of any one of SEQ ID NOs. 1 to 4.
[6] The vector according to any one of [1] to [5], wherein the vector is used in combination with a vector for expressing a capsid gene.
[7] The vector according to any one of [1] to [6], wherein the vector is used in combination with a vector for expressing a gene responsible for adenovirus helper function.
[8] A recombinant virus obtained from the vector according to any one of [1] to [7].
[9] A medicament comprising the vector according to any one of [1] to [7] or a recombinant virus obtained from the vector.
[10] The medicament according to [9], which is a medicament for a central nervous system disease.
[11] A method of introducing a gene into a microglial cell, the method including a step of introducing a target gene into the microglial cell in vitro, using the vector according to any one of [1] to [7].
[12] An isolated microglial cell into which the vector according to any one of [1] to [7] is introduced.
[13] A method of treating a central nervous system disease, the method including administering the vector according to any one of [1] to [7] or a recombinant virus obtained from the vector, to a patient in need thereof.
[14] The vector according to any one of [1] to [7] or a recombinant virus obtained from the vector, for use in the treatment of a central nervous system disease.
[15] Use of the vector according to any one of [1] to [7] or a recombinant virus obtained from the vector, in the production of a medicament for treating a central nervous system disease.

### EFFECT OF THE INVENTION

The present inventors have discovered that, in a microglia-specific AAV vector comprising an Iba1 promoter, sequences complementary to miRs and a WPRE gene, it is possible to increase the specificity of gene expression to microglia and to enhance the efficiency of gene expression as compared with the AAV vector in which sequences complementary to miRs are placed only downstream of the WPRE, by placing the sequences complementary to miRs upstream of the WPRE, or both upstream and downstream of the WPRE. In particular, the inventors discovered that an excellent microglial specificity is achieved when sequences complementary to miRs are placed on both sides of the WPRE. The vector according to the present invention enables the expression of a target protein in microglia specifically and efficiently.

### BRIEF DESCRIPTION OF THE DRAWINGS

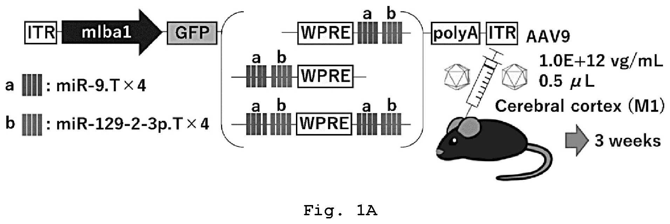

[FIG 1A] FIGs. 1A to 1F show the results of microglia-selective transduction in the cerebral cortex, by various types of AAV vectors incorporating an Iba1 promoter, sequences complementary to miRs, a WPRE gene and a GFP gene. The AAV9 vectors were injected into the M1 region of the cerebral cortex of C57BL/6J mice (0.5 µL; 1.0 E + 12 vg/mL). Three weeks after the injection, brain sections were immunostained for GFP and Iba1. FIG. 1A shows a schematic diagram of the DNA constructs of the various types of AAV vectors, and an administration scheme thereof in mice. Three patterns of the AAV vectors with different arrangements of the sequences complementary to miRs and the WPRE gene were examined. In other words, the arrangements are as follows: (1) the WPRE gene, the sequence complementary to miR-9, and the sequence complementary to miR-129-2-3p (the AAV vector with this arrangement is referred to as WPRE-a,b); (2) the sequence complementary to miR-9, the sequence complementary to miR-129-2-3p, and the WPRE gene (the AAV vector with this arrangement is referred to as a,b-WPRE); and (3) the sequence complementary to miR-9, the sequence complementary to miR-129-2-3p, the WPRE gene, the sequence complementary to miR-9, and the sequence complementary to miR-129-2-3p (the AAV vector with this arrangement is referred to as a,b-WPRE-a,b).

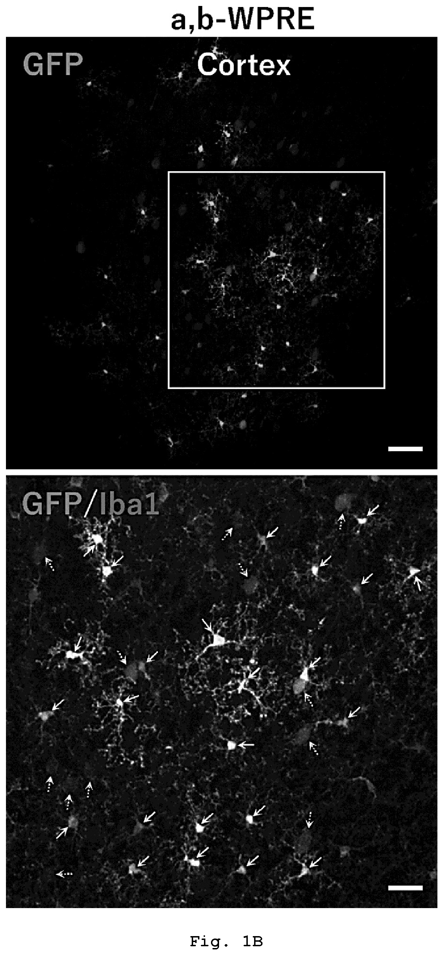

[FIG 1B] FIG. 1B shows photographs illustrating the results of immunohistochemical staining (GFP and Iba1) of the cerebral cortex in the case of injecting the a,b-WPRE. The lower photograph in FIG. 1B is an enlarged view of the region surrounded by the square in the upper photograph. The scale bars represent 50 µm (upper photograph) and 25 µm (lower photograph). The solid arrows indicate microglial cells, and the dashed arrows indicate neurons.

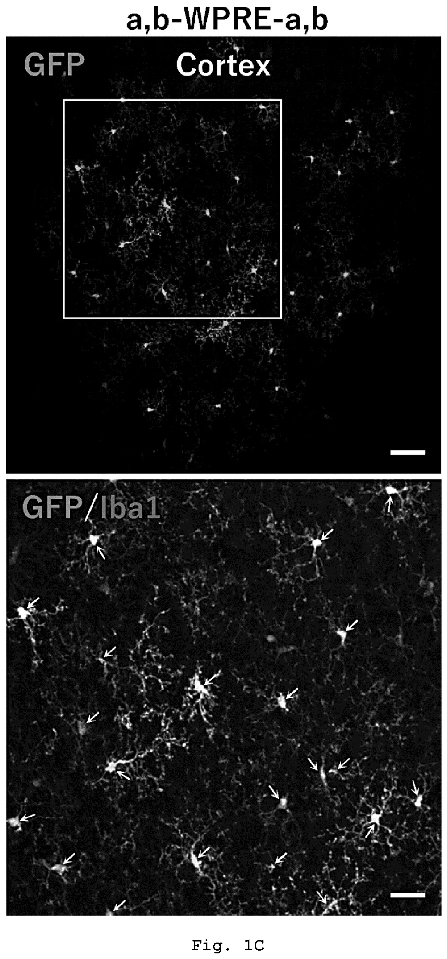

[FIG 1C] FIG. 1C shows photographs illustrating the results of immunohistochemical staining (GFP and Iba1) of the cerebral cortex in the case of injecting the a,b-WPRE-a,b. The lower photograph in FIG. 1C is an enlarged view of the region surrounded by the square in the upper photograph. The scale bars represent 50 µm (upper photograph) and 25 µm (lower photograph). The solid arrows indicate microglial cells.

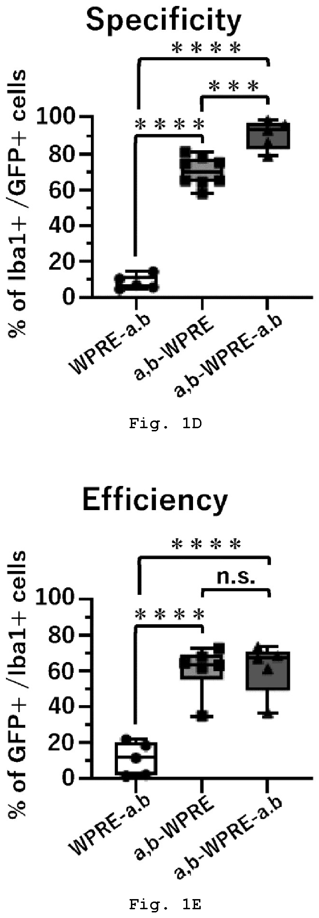

[FIG 1D] FIG. 1D is a graph showing microglial specificity when the various types of AAV vectors are used. The specificity is represented by the percentage (%) of Iba1-positive cells in GFP-positive cells. In FIG. 1D, the symbol "***" indicates p = 0.0005, and the symbol "****" indicates p < 0.0001.
[FIG 1E] FIG. 1E is a graph showing the expression efficiency in microglia when the various types of AAV vectors are used. The expression efficiency is represented by the percentage (%) of GFP -positive cells in Iba1-positive cells. In FIG. 1E, the symbol "****" indicates p < 0.0001, and the symbol "n.s." indicates that there is no significant difference.

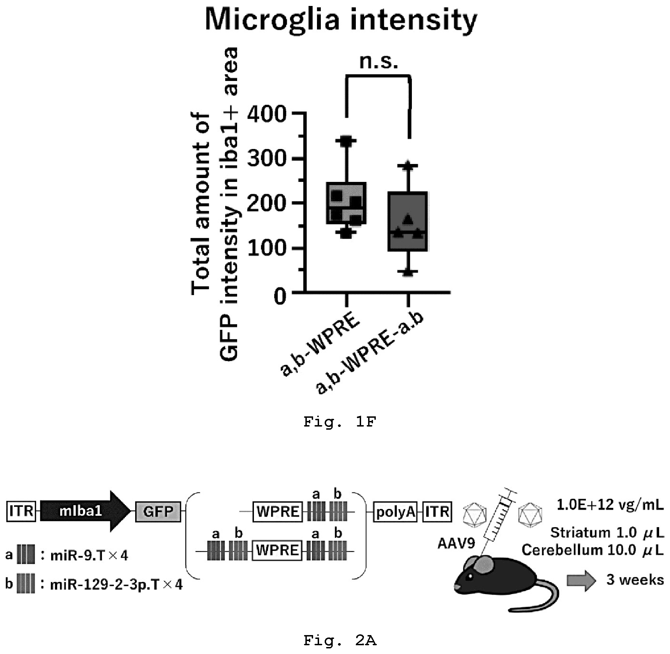

[FIG 1F] FIG. 1F is a graph showing the gene expression intensity in microglia when the various types of AAV vectors are used. The gene expression intensity in microglia is represented by the total GFP fluorescence intensity in Iba1-positive regions. In FIG. 1F, the symbol "n.s." indicates that there is no significant difference.
[FIG 2A] FIGs. 2A to 2G show the results of microglia-selective transduction in the striatum and the cerebellum, by various types of AAV vectors incorporating an Iba1 promoter, sequences complementary to miRs, a WPRE gene and a GFP gene. The AAV9 vectors were injected into the mouse striatum or the mouse cerebellum (in an amount of 1.0 µL or 10.0 µL; 1.0 E + 12 vg/mL). Three weeks after the injection, each brain section was immunostained for GFP and Iba1. FIG. 2A shows a schematic diagram of the DNA constructs of the various types of AAV vectors, and an administration scheme thereof in mice. Two patterns of the AAV vectors with different arrangements of the sequences complementary to miRs and the WPRE gene were examined. In other words, the arrangements are as follows: (1) the WPRE gene, the sequence complementary to miR-9, and the sequence complementary to miR-129-2-3p (the AAV vector with this arrangement is referred to as WPRE-a,b); and (2) the sequence complementary to miR-9, the sequence complementary to miR-129-2-3p, the WPRE gene, the sequence complementary to miR-9, and the sequence complementary to miR-129-2-3p (the AAV vector with this arrangement is referred to as a,b-WPRE-a,b).

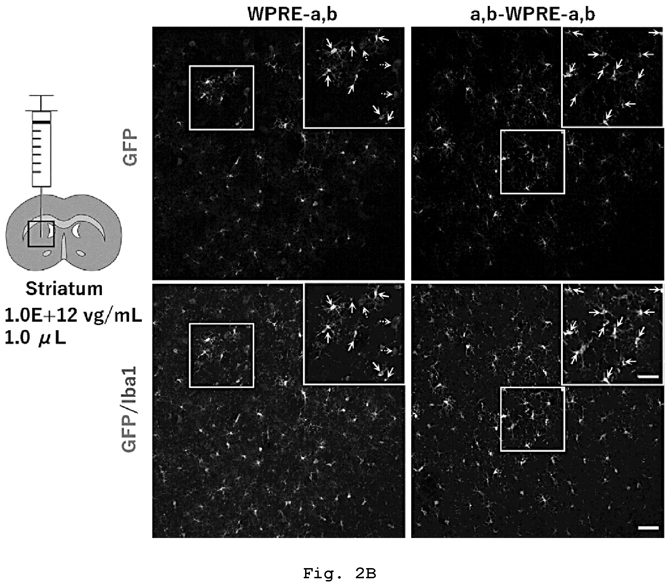

[FIG 2B] FIG. 2B shows photographs illustrating the results of immunohistochemical staining (GFP and Iba1) of the striatum, in the case of injecting WPRE-a,b or a,b-WPRE-a,b. The upper right photograph in each panel is an enlarged view of the region surrounded by the square in the panel. The scale bars represent 50 µm (each panel) and 25 µm (each enlarged view). The solid arrows indicate microglial cells, and the dashed arrows indicate neurons.

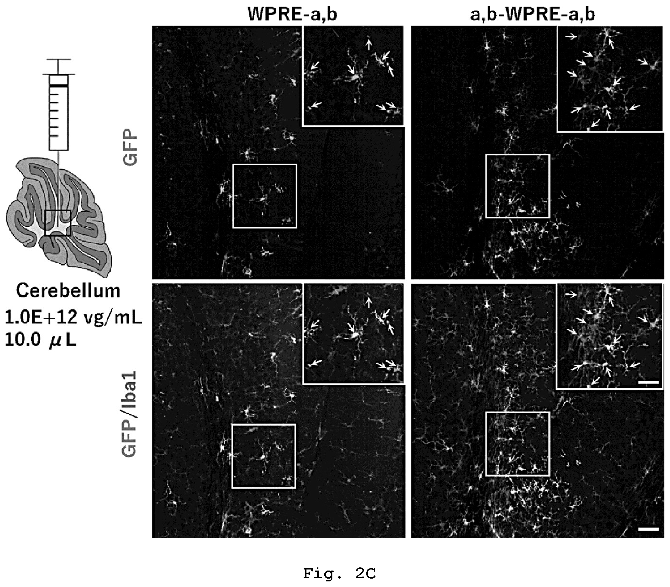

[FIG 2C] FIG. 2C shows photographs illustrating the results of immunohistochemical staining (GFP and Iba1) of the cerebellum, in the case of injecting WPRE-a,b or a,b-WPRE-a,b. The upper right photograph in each panel is an enlarged view of the region surrounded by the square in the panel. The scale bars represent 50 µm (each panel) and 25 µm (each enlarged view). The solid arrows indicate microglial cells.

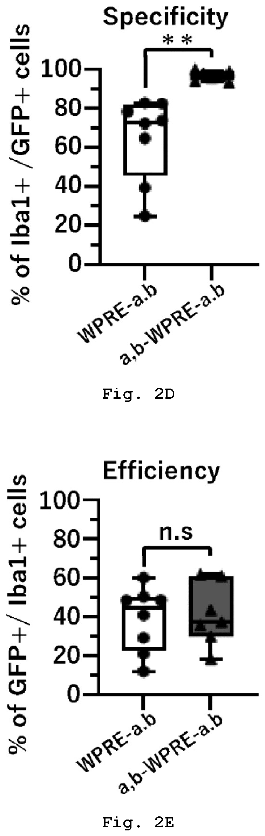

[FIG 2D] FIG. 2D is a graph showing microglial specificity in the striatum when the various types of AAV vectors are used. The specificity is represented by the percentage (%) of Iba1-positive cells in GFP-positive cells. In FIG. 2D, the symbol "**" indicates p = 0.0018.
[FIG 2E] FIG. 2E is a graph showing the expression efficiency in microglia in the striatum when the various types of AAV vectors are used. The expression efficiency is represented by the percentage (%) of GFP-positive cells in Iba1-positive cells. In FIG. 2E, the symbol "n.s." indicates that there is no significant difference.

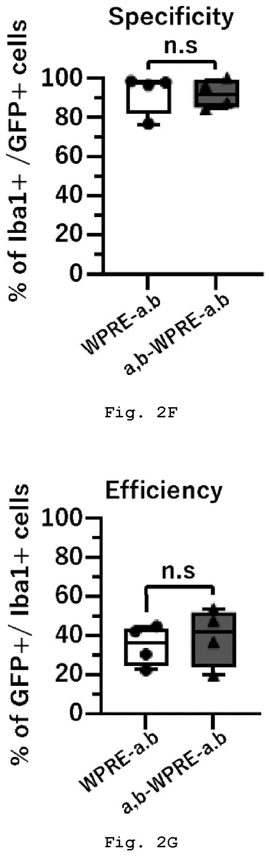

[FIG 2F] FIG. 2F is a graph showing microglial specificity in the cerebellum when the various types of AAV vectors are used. The specificity is represented by the percentage (%) of Iba1-positive cells in GFP-positive cells. In FIG. 2F, the symbol "n.s." indicates that there is no significant difference.
[FIG 2G] FIG. 2G is a graph showing the expression efficiency in microglia in the cerebellum when the various types of AAV vectors are used. The expression efficiency is represented by the percentage (%) of GFP-positive cells in Iba1-positive cells. In FIG. 2G, the symbol "n.s." indicates that there is no significant difference.

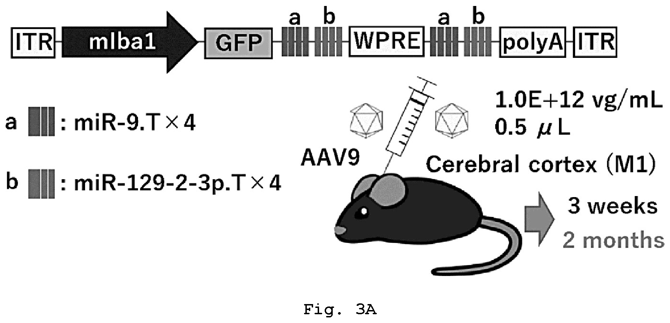

[FIG 3A] FIGs. 3A to 3F show the results of microglia-selective transduction in the cerebral cortex, by the AAV vector incorporating an Iba1 promoter, sequences complementary to miRs, a WPRE gene and a GFP gene. The arrangement of the sequences complementary to miRs and the WPRE gene is as follows: the sequence complementary to miR-9, the sequence complementary to miR-129-2-3p, the WPRE gene, the sequence complementary to miR-9, and the sequence complementary to miR-129-2-3p (the AAV vector with this arrangement is referred to as a,b-WPRE-a,b). The AAV9 vector was injected (0.5 µL; 1.0 E + 12 vg/mL) into the mouse cerebral cortex (M1 region). Three weeks after and two months after the injection, brain sections were immunostained for GFP and Iba1. FIG. 3A shows a schematic diagram of the DNA construct of the AAV vector, and an administration scheme thereof in mice.

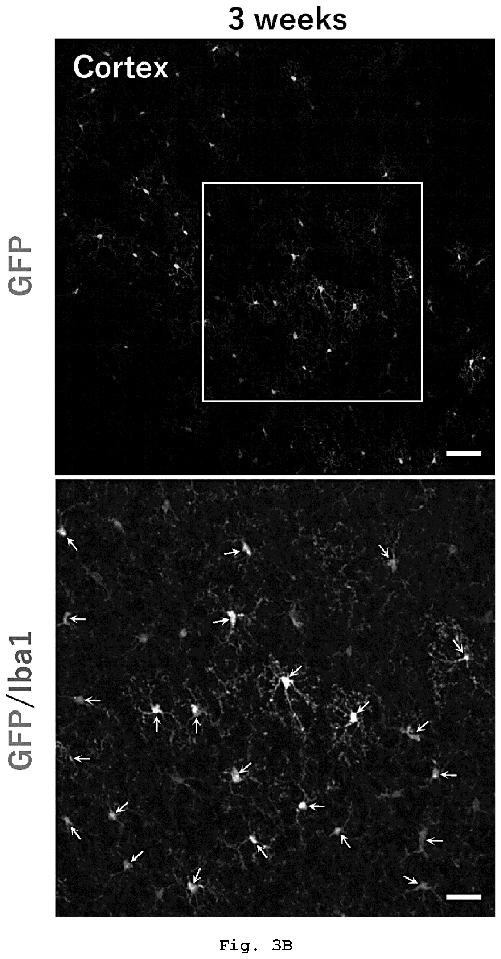

[FIG 3B] FIG. 3B shows photographs illustrating the results of immunohistochemical staining (GFP and Iba1) of the cerebral cortex three weeks after the injection of a,b-WPRE-a,b. The lower photograph in FIG. 3B is an enlarged view of the region surrounded by the square in the upper photograph. The scale bars represent 50 µm (upper photograph) and 25 µm (lower photograph). The solid arrows indicate microglial cells.

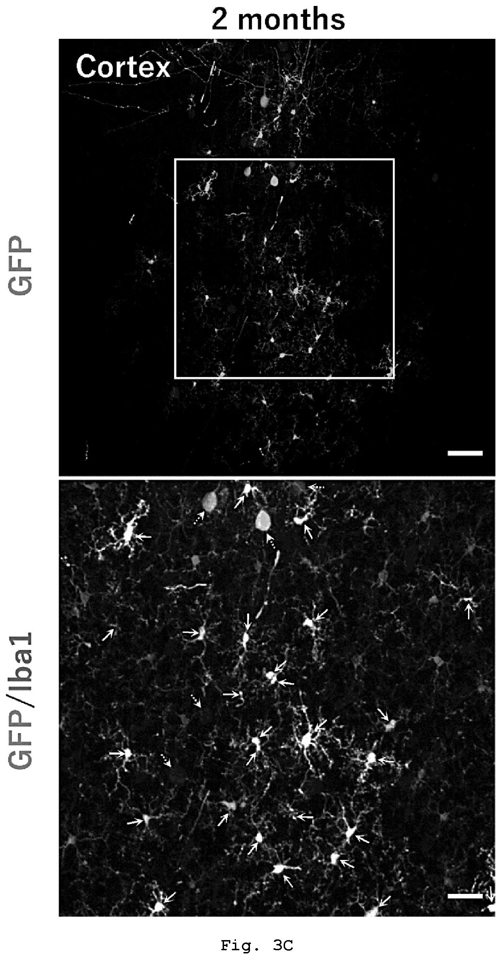

[FIG 3C] FIG. 3C shows photographs illustrating the results of immunohistochemical staining (GFP and Iba1) of the cerebral cortex two months after the injection of a,b-WPRE-a,b. The lower photograph in FIG. 3C is an enlarged view of the region surrounded by the square in the upper photograph. The scale bars represent 50 µm (upper photograph) and 25 µm (lower photograph). The solid arrows indicate microglial cells, and the dashed arrows indicate neurons.

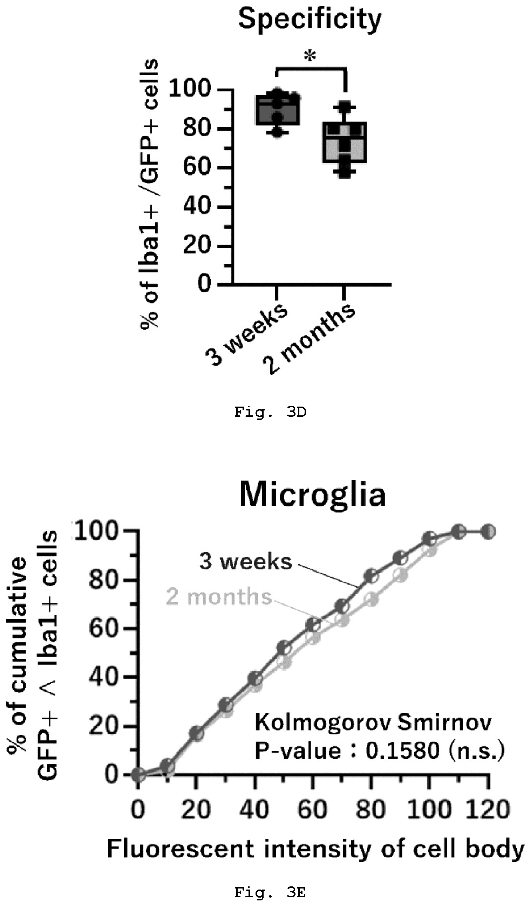

[FIG 3D] FIG. 3D is a graph showing microglial specificity in the cerebral cortex three weeks after and two months after the injection of the AAV vector. The specificity is represented by the percentage (%) of Iba1-positive cells in GFP-positive cells. In FIG. 3D, the symbol "*" indicates p = 0.0327.
[FIG 3E] FIG. 3E is a graph showing the cumulative percentage of microglia in the cerebral cortex three weeks after and two months after the injection of the AAV vector. The horizontal axis represents the fluorescence intensity in the cell bodies, and the vertical axis represents the cumulative percentage (%) of GFP-positive and Iba1-positive cells.

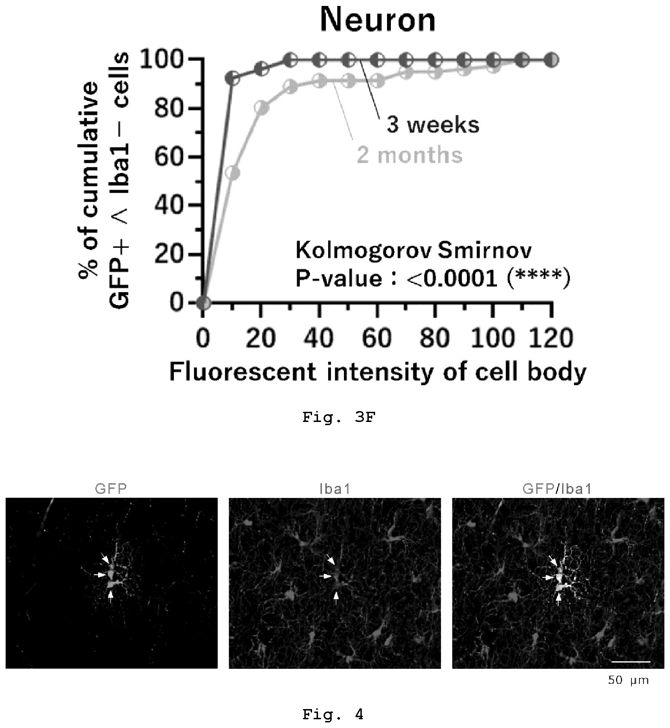

[FIG 3F] FIG. 3F is a graph showing the cumulative percentage of neurons in the cerebral cortex three weeks after and two months after the injection of the AAV vector. The horizontal axis represents the fluorescence intensity in the cell bodies, and the vertical axis represents the cumulative percentage (%) of GFP-positive and Iba1-negative cells.
[FIG 4] FIG. 4 shows the results of microglia-selective transduction in the marmoset cerebral cortex, by the AAV vector incorporating an Iba1 promoter, sequences complementary to miRs, a WPRE gene and a GFP gene. The AAV vector used is a,b-WPRE-a,b. Photographs illustrating the results of immunohistochemical staining (GFP and Iba1) of the cerebral cortex four weeks after the injection of the AAV vector are shown. The solid arrows indicate microglial cells.

### MODE FOR CARRYING OUT THE INVENTION

The present invention is an adeno-associated virus (AAV) vector for gene expression in microglia, the vector comprising an expression unit that comprises:
an Iba1 (ionized calcium-binding adaptor molecule 1) promoter,
a sequence complementary to miR-9,
a sequence complementary to miR-129, and
a woodchuck hepatitis virus post-transcriptional regulatory element (WPRE) gene,
wherein:
   the sequence complementary to miR-9 and the sequence complementary to miR-129 are placed upstream of the WPRE gene; or
   the sequence complementary to miR-9 and the sequence complementary to miR-129 are placed both upstream and downstream of the WPRE gene.

The term "upstream" refers to a region before (namely, on the 5' side of) a certain gene, and the term "downstream" refers to a region after (namely, on the 3' side of) a certain gene. A certain gene and a gene placed upstream or downstream thereof may be linked directly, or via one or a plurality of bases.

The "expression unit" of an AAV vector has inverted terminal repeat sequences (ITRs) at both ends, and the term can refer to the ITRs and the region sandwiched therebetween. The expression unit may further have a SV40 polyA sequence, and the SV40 polyA sequence may be placed upstream of the ITR at the end on the downstream side.

It is well known to those skilled in the art that there is a plurality of serotypes of adeno-associated viruses (AAVs). In the present invention, the serotype of AAV from which the AAV vector is derived is not limited, as long as the vector is capable of expressing a target gene in microglia. In other words, the portion of the vector other than the expression unit may be derived from any AAV. For example, AAVs of serotype 9, serotype 1, serotype 6 and the like can be used, and the AAV of serotype 9 (AAV9) is preferred. It is also possible to use a capsid variant. For example, a blood-brain barrier-penetrant capsid variant, or a capsid variant with tropism for microglia may be used in combination.

As the Iba1 promoter, any of the promoters of the Iba1 genes of mammals, such as humans, mice and rats, can be used, and it is possible to use a region of approximately 500 bp, 1 kbp, 1.5 kbp, 2 kbp or 3 kbp on the 5' side of the transcription start site in each Iba1 gene. The base sequence of the Iba1 promoter is not particularly limited. However, for example, a promoter having the base sequence of SEQ ID NO. 5 or 6 can be used. Alternatively, the Iba1 promoter may have only a partial sequence of the base sequence of SEQ ID NO. 5 or 6, or may have a sequence that is at least 90% identical to the base sequence of SEQ ID NO. 5 or 6, preferably has a sequence that is at least 95% identical thereto, and more preferably has a sequence that is at least 98% identical thereto, as long as a function as the Iba1 promoter can be exhibited.

In the case of using the vector according to the present invention for the purpose of treating a human, it is preferred to use a promoter having the base sequence of SEQ ID NO. 6.

The woodchuck hepatitis virus post-transcriptional regulatory element (WPRE) has the functions of preventing the read-through of the polyA site, promoting RNA processing and maturation, as well as enhancing RNA nuclear export. WPRE also has the function of acting on viral genome transcripts in packaging cells to promote vector packaging, thereby enhancing the viral titer. Further, WPRE has the function of enhancing the expression of the target gene in the transduced target cell, since WPRE promotes the maturation of mRNA produced by the internal promoter in the vector. The base sequence of the WPRE gene is not particularly limited. However, it is possible to use, for example, one having the base sequence of SEQ ID NO. 7.

The AAV vector according to the present invention can contain a target gene placed under the control of the Iba1 promoter, namely, a target gene linked to the Iba1 promoter.

The type of the target gene is not particularly limited. However, it is possible to use, for example, a marker gene such as a green fluorescent protein (GFP) gene (including a modified GFP), a Cre recombinase gene, luciferase, chloramphenicol acetyltransferase or lacZ; or a protein gene that detects the production of an intracellular second messenger such as Ca^{2 +} or cyclic AMP, and emits fluorescent light; a photoactivatable protein gene; or a toxin gene. For example, a GFP gene having the base sequence of SEQ ID NO. 8 can be used as the GFP gene.

Further, the target gene may also be a gene used for treating a central nervous system disorder. The gene having a therapeutic effect for a central nervous system disorder can be selected as appropriate depending on the type of the disease to be treated, and the like. In the case of multiple sclerosis, for example, it is possible to use a nucleic acid encoding a factor or element capable of inhibiting the inflammation of the brain parenchyma, or the like. It is also possible to use a nucleic acid encoding an anti-inflammatory cytokine that reduces brain damage, a nucleic acid encoding a brain-derived neurotrophic factor (BDNF) or a glial cell-derived neurotrophic factor (GDNF), or the like.

The AAV vector according to the present invention may be one in which such a target gene is placed under the control of the Iba1 promoter in advance, or may be one in which a multicloning site for incorporating such a target gene is placed downstream of the Iba1 promoter.

The vector according to the present invention comprises a sequence complementary to miR-9 and a sequence complementary to miR-129. The base sequences of both of these complementary sequences are not particularly limited, as long as the sequences are base sequences complementary to miR-9 and miR-129, respectively, which are present in the cells into which the vector according to the present invention is introduced. For example, the sequence complementary to miR-9 is the base sequence of SEQ ID NO. 9, and the sequence complementary to miR-129 is the base sequence of SEQ ID NO. 10. Alternatively, each of these sequences may have a sequence that is at least 90% identical to the base sequence of SEQ ID NO. 9 or 10, preferably has a sequence that is at least 95% identical thereto, and more preferably has a sequence that is at least 98% identical thereto, as long as the function thereof can be exhibited. The sequences complementary to the microRNA genes are used for degrading the mRNA transcribed from the AAV vector according to the present invention in cells other than microglia.

The vector according to the present invention infects not only microglia but also neurons and astrocytes as an AAV vector does, since the vector is a gene-modified vector constructed using an AAV vector as the basic structure.

The Iba1 promoter is a promoter having a high selectivity for microglia, and has a promoter activity on neurons and astrocytes as well. Therefore, miR-9.T and miR-129-2-3p.T are transcribed in neurons and astrocytes, by using the vector according to the present invention.

It is important to note here that miR-9 and miR-129-2-3p are endogenously present in neurons and astrocytes, but both of these microRNAs (miR-9 and miR-129-2-3p) are almost absent in microglia. In other words, even though exogenous genes (to which miR-9.T and miR-129-2-3p.T are added) introduced by the vector according to the present invention are transcribed in neurons and astrocytes, miR-9 and miR-129-2-3p that are endogenously present in neurons and astrocytes bind to miR-9.T and miR-129-2-3p.T contained in the mRNA transcribed from the AAV vector according to the present invention, and degrade the mRNA. In microglia, on the other side, miR-9 and miR-129-2-3p are absent, and thus the mRNA of the introduced exogenous genes is not degraded, allowing for the expression of the exogenous target gene.

The vector according to the present invention may contain only one copy each of the sequence complementary to miR-9 and the sequence complementary to miR-129, or alternatively, may contain multiple repeats of each of these sequences. The expression "to contain multiple repeats" may mean that the vector comprises, for example, from two to eight repeats of each of these sequences, preferably from two to six repeats of each of these sequences, and more preferably four repeats of each of these sequences, but not particularly limited thereto. The number of repeats of the sequence complementary to miR-9 and the number of repeats of the sequence complementary to miR-129 may be the same as, or different from, each other. The sequence complementary to miR-9 and the sequence complementary to miR-129 may be arranged alternately.

Further, the sequence complementary to miR-129 may be placed downstream of the sequence complementary to miR-9, and the sequence complementary to miR-9 may be placed downstream of the sequence complementary to miR-129. For example, four repeats of the sequence complementary to miR-129 may be placed downstream of four repeats of the sequence complementary to miR-9.

The vector according to the present invention may be one in which the sequence complementary to miR-9 and the sequence complementary to miR-129 are placed upstream of the WPRE gene, or may be one in which the sequence complementary to miR-9 and the sequence complementary to miR-129 are placed both upstream and downstream of the WPRE gene.

The order of arrangement of the genes incorporated into the AAV vector according to the present invention as well as the linker sequences between these genes are not particularly limited, as long as the genes are operably linked with each other. However, the AAV vector may contain, for example, a sequence in which the Iba1 promoter, the target gene, the sequence complementary to miR-9 (repeated four times), the sequence complementary to miR-129 (repeated four times) and the WPRE gene are arranged in this order from the 5' side. Alternatively, the vector may contain a sequence in which the Iba1 promoter, the target gene, the sequence complementary to miR-9 (repeated four times), the sequence complementary to miR-129 (repeated four times), the WPRE gene, the sequence complementary to miR-9 (repeated four times), the sequence complementary to miR-129 (repeated four times) are arranged in this order from the 5' side.

The vector according to the present invention may contain a selection marker. The "selection marker" refers to a genetic element that provides a selectable phenotype to the cell into which the selection marker is introduced. In general, the selection marker is a gene whose gene product imparts resistance to a drug that inhibits the growth of cells or kills cells. Specific examples of the selection marker include Neo gene, Hyg gene, hisD gene, Gpt gene, and Ble gene. Examples of drugs useful for selecting the presence of the selection marker include G418 for Neo, hygromycin for Hyg, histidinol for hisD, xanthine for Gpt, and bleomycin for Ble.

The vector according to the present invention may contain another functional gene, such as, for example, an enhancer.

The vector according to the present invention is, for example, a vector comprising a base sequence of any one of SEQ ID NOs. 1 to 4, but not particularly limited thereto. Each of the base sequences of SEQ ID NO. 1 and 2 comprises a sequence in which the Iba1 promoter, the target gene, the sequence complementary to miR-9 (repeated four times), the sequence complementary to miR-129 (repeated four times) and the WPRE gene are arranged in this order from the 5' side. The base sequence of SEQ ID NO. 1 is a sequence having two recognition sequences for the restriction enzyme NotI, and the base sequence of SEQ ID NO. 2 is a sequence having one recognition sequence for the restriction enzyme NotI. Further, each of the base sequences of SEQ ID NOs. 3 and 4 comprises a sequence in which the Iba1 promoter, the target gene, the sequence complementary to miR-9 (repeated four times), the sequence complementary to miR-129 (repeated four times), the WPRE gene, the sequence complementary to miR-9 (repeated four times), the sequence complementary to miR-129 (repeated four times) are arranged in this order from the 5' side. The base sequence of SEQ ID NO. 3 is a sequence having two recognition sequences for the restriction enzyme NotI, and the base sequence of SEQ ID NO. 4 is a sequence having one recognition sequence for the restriction enzyme NotI.

The vector according to the present invention may be a vector consisting of the base sequence of any one of SEQ ID NOs. 1 to 4, or may be a vector further comprising a base sequence other than the base sequence of any one of SEQ ID NOs. 1 to 4. Alternatively, the present vector may be a vector comprising a base sequence having a sequence that is at least 90% identical to the base sequence of any one of SEQ ID NOs. 1 to 4, preferably at least 95% identical thereto, and more preferably at least 98% identical thereto, as long as the promoter, the target gene and the sequences complementary to miRs function.

The length of the expression unit is usually up to about 4.7 kbp, and the length of the entire AAV is up to about 7.5 kbp.

Incorporation of any of the various types of genes described above into an AAV vector sequence can be performed by a method known to those skilled in the art. For example, any of the various types of genes can be incorporated into the vector sequence by a method using a restriction enzyme treatment, but not particularly limited thereto.

A method using a known AAV vector can be used as a gene transfer method. Specifically, a pAAV plasmid, a pRC plasmid and a helper plasmid are introduced into a packaging cell, such as an HEK293 cell, to form a virus, and the target brain cells, preferably glial cells, more preferably microglial cells are infected with the resulting virus.

The vector according to the present invention may be used in combination with a vector for expressing a capsid. Examples of the capsid include AAV9 capsid, AAV1 capsid, and AAV6 capsid. For example, a pAAV derived from serotype 2 may be used, with AAV9 selected as the capsid.

The vector according to the present invention may be used in combination with a vector for expressing a gene responsible for adenovirus helper function.

The gene responsible for adenovirus helper function is not particularly limited as long as the gene is responsible for adenovirus helper function, and examples thereof include E1A, E1B, E2A, E2B, E3, E4, and VA. One or a plurality of these genes may be included.

The helper function is not particularly limited as long as it is known to those skilled in the art. For example, E1A has the function of enhancing transcriptional activity, and the like; E1B has the function of controlling apoptosis, and the like; and E2A and E2B have the function of replicating DNA, and the like.

Another aspect of the present invention is a recombinant virus obtained from any one of the vectors described above.

Yet another aspect of the present invention is a medicament comprising any one of the vectors described above or a recombinant virus obtained therefrom.

The medicament described above is, for example, a gene therapy drug for the treatment of a central nervous system disease or a disease that causes abnormalities in microglia. The medicament enables the introduction of a target gene into microglial cells in the cerebral cortex, the striatum, the cerebellum, and the like, specifically and efficiently.

The disease to be treated is not particularly limited, as long as the disease is a central nervous system disease, and a disease in which microglia are involved. Examples of the central nervous system disease include: neurodegenerative diseases such as Alzheimer's disease, Parkinson's disease and spinocerebellar ataxia; neuroinflammation such as multiple sclerosis; and mental illness.

The content of the active ingredient in the medicament as well as the dosage and administration of the medicament are not particularly limited as long as the therapeutic effect can be obtained, and can be determined as appropriate depending on the type of the disease, the degree of the disease, patient age and body weight, the expression efficiency and/or the like.

The medicament according to the present invention may contain another active ingredient. The medicament can also be used, for example, in combination with another therapeutic agent for a central nervous system disease.

Yet another aspect of the present invention is a method of introducing any one of the vectors described above into a microglial cell in vitro. However, the method includes a method of introducing any one of the vectors described above into microglia in a non-human animal in vivo, as well. The microglia may be derived from any of the cerebral cortex, the striatum, and the cerebellum.

The method of introducing the vector into a microglial cell is not particularly limited. However, it is preferred to use a method based on the infectivity inherent in the AAV vector.

Yet another aspect of the present invention is an isolated microglial cell into which any one of the vectors described above is introduced.

Other aspects of the present invention may be, for example, as follows:
· A method of treating a central nervous system disease, the method including administering the vector according to any one of the above or a recombinant virus obtained from the vector, to a patient in need thereof.
· The vector according to any one of the above or a recombinant virus obtained from the vector, for use in the treatment of a central nervous system disease.
· Use of the vector according to any one of the above or a recombinant virus obtained from the vector, in the production of a medicament for treating a central nervous system disease.

### EXAMPLES

The present invention will now be described in further detail. However, it goes without saying that the scope of the present invention is not limited only to the Examples.

### <Vector Construction>

The vector according to the present invention was constructed by the same vector construction method as that described in Patent Document 1. A sequence was designed based on the microRNA sequences obtained from the miRNA Registry (www.mirbase.org). To construct the vector, sense 1 to sense 4 (S1 to S4, SEQ ID NOs. 11 to 14) and antisense 1 to antisense 4 (AS1 to AS4, SEQ ID NOs. 15 to 18), as oligonucleotides, were annealed, and inserted into the restriction enzyme sites in a gene expression cassette of an AAV-PGK vector, using a method involving restriction enzyme treatment known to those skilled in the art.

The genomic region of 1,678 bp upstream (5' side) of the first ATG in exon 1 of the mouse Iba1 gene was used as the Iba1 promoter. The Iba1 promoter region was amplified from genomic DNA derived from mouse brain cells by two-step (nested) PCR. In the first step of DNA amplification, Iba1-nest-F (5'-CCTAGAGCCATCTTGTAAGG-3', SEQ ID NO. 19) and Iba1-nest-R (5'-CGAGGAATTGCTGTTGAG-3', SEQ ID NO. 20) were used; and in the second step of DNA amplification, Iba1-F (5'-ATGCTCTAGActcgagTACTATAGGATGCATCGTGAAAACC-3' SEQ ID NO. 21) and Iba1-R (5'-CATGGTGGCGaccggtGGCTCCTCAGACGCTGGTTG-3', SEQ ID NO. 22) were used.

The vector comprising an Iba1 promoter according to the present invention was obtained by replacing the PGK promoter in the AAV-PGK vector constructed as described above, with the Iba1 promoter. In other words, the PGK promoter in the corresponding AAV-PGK vector was replaced with a cloned 1.7 kb fragment of the mouse Iba1 promoter by cleaving at the XhoI and AgeI restriction enzyme sites.

### <Preparation of AAV9 Vector>

HEK293 T cells were simultaneously transfected with three plasmids, namely, a pRC expression plasmid, a pHelper plasmid (Stratagene, La Jolla, CA, USA) and a pAAV9 plasmid, to prepare AAV9 vector particles. The virus particles were precipitated with ammonium sulfate or polyethylene glycol 8000, and purified by continuous density gradient centrifugation with iodixanol. The genomic titer of the purified AAV9 vector was determined by quantitative real-time PCR targeting the WPRE sequence, using a PowerSYBR (registered trademark) Green PCR Master Mix (Thermo Fisher Scientific, Inc), and using, as primers, 5'-CTGTTGGGCACTGACAATTC-3' (SEQ ID NO. 23) and 5'-GAAGGGACGTAGCAGAAGGA-3' (SEQ ID NO. 24).

### <Animals>

C57BL/6J mice (Examples 1 to 3) and a marmoset (Example 4) were used in the present study. All animal procedures were conducted in accordance with protocols approved by the Japanese Act on the Welfare and Management of Animals and the Guidelines for Proper Conduct of Animal Experiments issued by the Science Council of Japan.

### <Stereotactic Injection of AAV9 Vector into Brain Parenchyma>

In Examples 1 to 3, the mice were anesthetized by intraperitoneal injection of ketamine (100 mg/body weight (kg)) and xylazine (10 mg/body weight (kg)). The depth of anesthesia was monitored by toe pinch reflex throughout the operation, and additional ketamine and xylazine were injected if necessary. A burr hole was drilled above the injection site to expose the brain. The AAV vector according to the present invention was injected into the cerebral cortex, the striatum and the cerebellum of each mouse. A 10 µL Hamilton syringe with a 33 G needle was used in order to reduce the injection volume and increase the accuracy. The following stereotactic coordinates were used for the injection of the viral vector: cerebral cortex, AP -1.0 mm, ML +1.0 mm, DV +0.8 mm; striatum, AP -1.0 mm, ML +1.75 mm, DV +2.75 mm; cerebellum, AP +6.5 mm, ML 0 mm, DV +1.8 mm (all values are relative to the bregma). The volume and the injection rate of the AAV (titer: 1.0 E + 12 vg/mL) injected into the brain of each mouse are: 0.5 µL and 25 nL/min in the cerebral cortex, respectively; 1 µL and 50 nL/min in the striatum, respectively; and 10 µL and 500 nL/min in the cerebellum, respectively. The stereotactic injection into the marmoset cerebral cortex in Example 4 was also carried out in a manner similar to that described above. However, 1 µL (5 × 10⁹ vg) of the AAV9 vector with a titer of 5 × 10¹² vg/mL was injected to the marmoset cerebral cortex.

### <Immunohistochemistry>

In Examples 1 to 3, the mice were perfused transcardially with 0.1 M PBS (pH 7.4) containing 4% paraformaldehyde, the harvested brain was subsequently fixed overnight, and then transferred into a 1 × PBS solution. The brain was cut into coronal slices (the cerebral cortex and striatum) or sagittal slices (the cerebellum) with a thickness of 50 µm, using a microtome (VT1000S; Leica, Germany). The slices were allowed to react overnight at 4°C with primary antibodies: rat anti-GFP (Nakalai Tesque, Inc.) 1:1,000, and rabbit anti-Iba1 (Wako Pure Chemical Industries, Ltd.) 1:500, which were mixed with a blocking solution (5% normal donkey serum, 0.5% Triton X-100 and 0.05% NaN₃ in PBS). After washing five times with PBS, the slices were immersed in PBS and shaken for 15 minutes. Thereafter, the slices were incubated at room temperature for three hours with anti-IgG secondary antibodies: Alexa Fluor Plus donkey anti-rat 488 and Alexa Fluor Plus donkey anti-rabbit 555, which were mixed with the blocking solution at a ratio of 1:2,000. Finally, the slices were washed five times with PBS, then immersed in PBS and shaken for 15 minutes, and thereafter, mounted with a ProLong Diamond Antifade Mountant (Thermo Fisher Scientific, Inc.). Immunohistochemistry of the marmoset cerebral cortex in Example 4 was also carried out in a manner similar to that described above. However, an anti-GFP antibody (rat, 1:1,000, 04404-84; Nakalai Tesque, Inc.) and anti-Iba1 antibody (rabbit, 1:500, 019-19741; FUJIFILM Wako Pure Chemical Corporation) were used as primary antibodies, and a ProLong Glass Antifade Mountant with NucBlue Stain (P36983; Thermo Fisher Scientific, Inc.) was used as the mountant, for the marmoset cerebral cortex.

### <Confocal Laser Microscopy Analysis>

In Examples 1 to 3, the proportion of the cells transduced by the AAV vector was examined by immunohistochemistry. Fifty µm thick sections were prepared from the cerebral cortex, the striatum and the cerebellum, and the sections were double immunostained with antibodies against GFP and Iba1. Regions in which GFP fluorescence can be observed were photographed with a confocal laser scanning microscope (LSM800; Carl Zeiss AG, Oberkochen, Germany) at regular intervals with its optimal settings, using a 10×/0.95 NA or 20×/0.95 NA objective lens. Subsequently, image processing software (ZEN blue) was used to perform maximum-intensity projection (MIP). MIP images were captured from each brain slice so that the imaging fields did not overlap, and the number of GFP-expressing cells was counted. GFP-positive cells co-labeled with Iba1 were regarded as microglial cells. At least 30 cells were counted. The confocal laser microscopy analysis of the marmoset cerebral cortex in Example 4 was also carried out in a manner similar to that described above. However, 100 µm-thick sections were prepared from the marmoset cerebral cortex, and the sections were photographed with an all-in-one fluorescence microscope (BZ-X800, manufactured by Keyence Corporation).

### <Statistical Analysis>

Statistical analyses for the specificity of gene expression, the expression efficiency, as well as the fluorescence intensity in FIG. 1F, were carried out by the unpaired Student's t-test or the one-way ANOVA test, and statistical analyses for FIGs. 3E and 3F were carried out by generating graphs using cumulative plots and performing the Kolmogorov-Smirnov test.

### <Example 1: AAV Vector in Which Sequences Complementary to miRs Are Placed Both Upstream and Downstream of WPRE Gene Shows High Microglial Specificity in Cerebral Cortex>

DNA constructs of various types of AAV vectors comprising an Iba1 promoter, sequences complementary to miRs, a WPRE gene and the like were prepared by the method described above (FIG. 1A). The following three patterns of the AAV vectors with different arrangements of the sequences complementary to miRs and the WPRE gene were prepared. Each of the sequence complementary to miR-9 and the sequence complementary to miR-129-2-3p was repeated four times. The arrangements are as follows:
(1) the WPRE gene, the sequence complementary to miR-9, and the sequence complementary to miR-129-2-3p (the AAV vector with this arrangement is referred to as WPRE-a,b (control));
(2) the sequence complementary to miR-9, the sequence complementary to miR-129-2-3p, and the WPRE gene (the AAV vector with this arrangement is referred to as a,b-WPRE); and
(3) the sequence complementary to miR-9, the sequence complementary to miR-129-2-3p, the WPRE gene, the sequence complementary to miR-9, and the sequence complementary to miR-129-2-3p (the AAV vector with this arrangement is referred to as a,b-WPRE-a,b).

Various types of AAV vectors were injected into the mouse cerebral cortex. Three weeks after the injection, brain slices were prepared, and the brain slices were analyzed immunohistochemically by double immunolabeling with antibodies against GFP and Iba1.

As a result, microglia expressing GFP and Iba1 were observed regardless of whether a,b-WPRE (FIG. 1B) or a,b-WPRE-a,b (FIG. 1C) was used. Further, it has been shown that the microglial specificity and the expression efficiency in microglia are significantly higher regardless of whether a,b-WPRE or a,b-WPRE-a,b is used, as compared with the case of using WPRE-a,b, which served as the control AAV vector (FIG. 1D to E). In addition, it has been shown that the microglial specificity is significantly higher when a,b-WPRE-a,b is used, as compared with the case of using a,b-WPRE (FIG. 1E). The gene expression intensity in microglia in the case of using a,b-WPRE was about the same as that in the case of using a,b-WPRE-a,b (FIG. 1F).

These results have revealed that a high microglial specificity and a high expression efficiency in microglia are achieved in the gene expression in the cerebral cortex, by using the AAV vector in which the sequences complementary to miRs are placed upstream of the WPRE gene or both upstream and downstream of the WPRE gene. Further, the results have shown that the AAV vector in which the sequences complementary to miRs are placed both upstream and downstream of the WPRE gene, among such AAV vectors, is particularly excellent in enhancing the microglial specificity.

### <Example 2: AAV Vector in Which Sequences Complementary to miRs Are Placed Both Upstream and Downstream of WPRE Gene Shows High Microglial Specificity, Also in Other Brain Sites>

It was examined whether or not the AAV vector in which the sequences complementary to miRs are placed both upstream and downstream of the WPRE gene shows a high microglial specificity in brain sites (the striatum and cerebellum) other than the cerebral cortex.

Among the AAV vectors used in Example 1, the following two types of vectors were used (FIG. 2A).

### (1) WPRE-a,b (control)

### (2) a,b-WPRE-a,b

Various types of AAV vectors were injected into the striatum and the cerebellum of the mice. Three weeks after the injection, brain slices were prepared, and the brain slices were analyzed immunohistochemically by double immunolabeling with antibodies against GFP and Iba1.

As a result, microglia expressing GFP and Iba1 were observed in both the striatum (FIG. 2B) and the cerebellum (FIG. 2C), regardless of whether WPRE-a,b or a,b-WPRE-a,b was used. In the striatum, it has been shown that the microglial specificity is significantly higher in the case of using a,b-WPRE-a,b, as compared with the case of using WPRE-a,b, which served as the control AAV vector (FIG. 2D). In the cerebellum, it has been shown that a sufficiently high microglial specificity of 90% or more is achieved even in the case of using WPRE-a,b, which served as the control AAV vector, but a similarly high specificity is achieved also in the case of using a,b-WPRE-a,b (FIG. 2F). Further, in both the striatum and the cerebellum, no significant difference was observed in the expression efficiency in microglia between the case of using WPRE-a,b, which served as the control AAV vector, and the case of using a,b-WPRE-a,b (FIG. 2E and FIG. 2G).

These results have revealed that a high microglial specificity is achieved in the gene expression in brain sites (the striatum and cerebellum) other than the cerebral cortex as well, by using the AAV vector in which the sequences complementary to miRs are placed both upstream and downstream of the WPRE gene.

### <Example 3: AAV Vector in Which Sequences Complementary to miRs Are Placed Both Upstream and Downstream of WPRE Gene Continues to Show High Microglial Specificity over Long Period of Time (Two Months)>

It was examined whether or not the AAV vector in which the sequences complementary to miRs are placed both upstream and downstream of the WPRE gene shows a high microglial specificity in the cerebral cortex over a long period of time.

Among the AAV vectors used in Example 1, a,b-WPRE-a,b was used (FIG. 3A).

The AAV vector was injected into the mouse cerebral cortex. Three weeks after and two months after the injection, brain slices were prepared, and the brain slices were analyzed immunohistochemically by double immunolabeling with antibodies against GFP and Iba1.

As a result, microglia expressing GFP and Iba1 were observed both three weeks after (FIG. 3B) and two months after (FIG. 3C) the injection of the AAV vector. Further, in the case of using a,b-WPRE-a,b, it has been shown that, although the microglial specificity decreases to some extent with the passage of time, the microglial specificity is high at both time points three weeks after and two months after the injection of the AAV vector (FIG. 3D). In addition, it has been shown that there is no significant difference in the cumulative percentage of microglia in the cerebral cortex between the time points of three weeks after and two months after the injection of the AAV vector (FIG. 3E), and that there is a significant difference in the cumulative percentage of neurons therein between both time points (FIG. 3F).

These results have revealed that a high microglial specificity is achieved in the gene expression in the cerebral cortex over a long period of time of at least two months, by using the AAV vector in which the sequences complementary to miRs are placed both upstream and downstream of the WPRE gene.

Microglia are known to be deeply involved in the pathological conditions of central nervous system diseases. However, since it has been impossible to efficiently express exogenous genes in a microglia-selective manner so far, gene therapy targeting microglia has not been performed, and studies with gene therapy in mind have made almost no progress. It is expected that the use of the AAV vector according to the present invention will lead to the widespread use of gene therapy targeting microglia in the future.

### <Example 4: AAV Vector in Which Sequences Complementary to miRs Are Placed Both Upstream and Downstream of WPRE Gene Shows Its Capacity to Express Target Gene in Microglia in Cerebral Cortex of Primate>

It was examined whether or not the AAV vector in which the sequences complementary to miRs are placed both upstream and downstream of the WPRE gene is capable of expressing a target gene in microglia in the cerebral cortex, even in a primate.

Among the AAV vectors used in Example 1, a,b-WPRE-a,b was used. One µL (5 × 10⁹ vg) of the AAV9 vector with a titer of 5 × 10¹² vg/mL was injected into the cerebral cortex of a marmoset (individual ID H292: Izumo, 1.6 years old, body weight: 509 g). Twenty-nine days later (four weeks later), the marmoset was sacrificed, and the brain was harvested. The body weight at the time of sacrifice was 532 g. Brain slices with a thickness of 100 µm were prepared, the slices were fluorescently immunostained with an anti-GFP antibody and an anti-Iba1 antibody, and then photographed with an all-in-one fluorescence microscope (BZ-X800, manufactured by Keyence Corporation).

As a result, microglia expressing GFP and Iba1 were observed. In other words, it has been confirmed that a target gene can be expressed in microglia in the cerebral cortex, even in a primate, by using the AAV vector in which the sequences complementary to miRs are placed both upstream and downstream of the WPRE gene.

## Claims

1. An adeno-associated virus (AAV) vector for gene expression in microglia, the vector comprising an expression unit that comprises
an Iba1 (ionized calcium-binding adaptor molecule 1) promoter,
a sequence complementary to miR-9,
a sequence complementary to miR-129, and
a woodchuck hepatitis virus post-transcriptional regulatory element (WPRE) gene,
wherein
the sequence complementary to miR-9 and the sequence complementary to miR-129 are placed upstream of the WPRE gene, or
the sequence complementary to miR-9 and the sequence complementary to miR-129 are placed both upstream and downstream of the WPRE gene.

2. The vector according to claim 1, further comprising a target gene placed under the control of the Iba1 promoter.

3. The vector according to claim 2, wherein the target gene is a green fluorescent protein (GFP) gene.

4. The vector according to claim 1, wherein the vector comprises multiple repeats of each of the sequence complementary to miR-9 and the sequence complementary to miR-129.

5. The vector according to claim 1, wherein the vector comprises a base sequence of any one of SEQ ID NOs. 1 to 4, or a base sequence having a sequence that is at least 90% identical to the base sequence of any one of SEQ ID NOs. 1 to 4.

6. The vector according to claim 1, wherein the vector is used in combination with a vector for expressing a capsid gene.

7. The vector according to claim 1, wherein the vector is used in combination with a vector for expressing a gene responsible for adenovirus helper function.

8. A recombinant virus obtained from the vector according to any one of claims 1 to 7.

9. A medicament comprising the vector according to any one of claims 1 to 7 or a recombinant virus obtained from the vector.

10. The medicament according to claim 9, which is a medicament for a central nervous system disease.

11. A method of introducing a gene into a microglial cell, the method comprising a step of introducing a target gene into the microglial cell in vitro, using the vector according to any one of claims 1 to 7.

12. An isolated microglial cell into which the vector according to any one of claims 1 to 7 is introduced.
